(19) Europäisches Patentamt
European Patent Office
Office européen des brevets

(11) Veröffentlichungsnummer: **0 014 910**
**B1**

(12) # EUROPÄISCHE PATENTSCHRIFT

(45) Veröffentlichungstag der Patentschrift: **29.06.83**

(51) Int. Cl.³: **A 61 K 31/23**, A 23 L 1/34

(21) Anmeldenummer: **80100633.9**

(22) Anmeldetag: **07.02.80**

(54) **Vollständig acetylierte Monoglyceride zur Verwendung zur Senkung von überhöhten Cholesterinspiegeln und/oder Neutralfettspiegeln im Blut von Menschen und Verfahren zur Herstellung von pharmazeutischen Produkten für diesen Zweck und diese enthaltende diätetische Nahrungsmittel.**

(30) Priorität: **16.02.79 DE 2905979**

(43) Veröffentlichungstag der Anmeldung:
**03.09.80 Patentblatt 80/18**

(45) Bekanntmachung des Hinweises auf die Patenterteilung:
**29.06.83 Patentblatt 83/26**

(84) Benannte Vertragsstaaten:
**AT BE CH FR GB IT LU NL SE**

(56) Entgegenhaltungen:
**DE - B - 1 076 115**
**FR - A - 1 155 903**
**FR - A - 1 206 623**
**FR - M - 3 598**
**US - A - 2 615 159**
**US - A - 3 027 259**
**US - A - 3 689 514**

(73) Patentinhaber: **Gatzen, Carl-Jacob**
**Pannebäckerstrasse 20**
**D-4000 Düsseldorf 13 (DE)**

(73) Patentinhaber: **Poethen, Hartmut, Dr. med.**
**Berger Allee 3**
**D-4000 Düsseldorf (DE)**

(73) Patentinhaber: **Sion, Heribert, Dr.med.**
**Graf-Adolf-Platz 3**
**D-4000 Düsseldorf 1 (DE)**

(72) Erfinder: **Gatzen, Carl-Jacob**
**Pannebäckerstrasse 20**
**D-4000 Düsseldorf 13 (DE)**
Erfinder: **Poethen, Hartmut, Dr. med.**
**Berger Allee 3**
**D-4000 Düsseldorf (DE)**
Erfinder: **Sion, Heribert, Dr.med.**
**Graf-Adolf-Platz 3**
**D-4000 Düsseldorf 1 (DE)**

(74) Vertreter: **Redies, Bernd, Dr. rer. nat. et al,**
**Redies, Redies, Türk & Gille Brucknerstrasse 20**
**D-4000 Düsseldorf 13 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 014 910 B1

# Vollständig acetylierte Monoglyceride zur Verwendung zur Senkung von überhöhten Cholesterinspiegeln und/oder Neutralfettspiegeln im Blut von Menschen und Verfahren zur Herstellung von pharmazeutischen Produkten für diesen Zweck und diese enthaltende diätetische Nahrungsmittel

Die vorliegende Erfindung betrifft an sich bekannte, vollständig acetylierte, destillierte, z.B. und insbesondere aus Schweineschmalz, Baumwollsamenöl oder teilweise hydriertem Pflanzenöl hergestellte Monoglyceride oder Gemische aus mehreren solcher Monoglyceride zur Behandlung von Störungen im Bereich des Fettstoffwechsels beim Menschen und insbesondere zur Verwendung zur Senkung von überhöhten Cholesterinspiegeln und/oder Neutralfettspiegeln im Blut von Menschen und/oder zur Überwindung von Therapieresistenz von Menschen mit erhöhtem Cholesterin- und/oder Neutralfettblutspiegel gegen übliche pharmazeutische Produkte zur Senkung von überhöhtem Cholesterin- und/oder Neutralfettspiegel im Blut von Menschen.

Die Erfindung betrifft weiterhin ein oder mehrere solcher Monoglyceride enthaltende oder hieraus bestehende pharmazeutische Zubereitungen zu dem genannten Zweck. Weiterhin betrifft die Erfindung ein oder mehrere solcher Monoglyceride enthaltende diätetische Nahrungsmittel, die zu dem gleichen Zweck eingesetzt werden und eine leicht handzuhabende Verabreichungsform für die erfindungsgemäß zu verwendenden Monoglyceride darstellen.

Ein hoher Cholesterinspiegel im Blut des Menschen ist die Folge von Störungen des Fettstoffwechsels. Derart erhöhte Cholesterinspiegel sind nach derzeitigen Erkenntnissen der Wissenschaft Ursache für Arteriosklerose und Hypertonie, welch letztere wieder mitursächlich für Anfälligkeit gegen Herzinfarkt und andere schwerwiegende Kreislaufstörungen oder Diabetes oder Störungen der Schilddrüsenfunktion ist. Auch überhöhte Blutfettwerte, die häufig, aber nicht stets mit überhöhten Cholesterinspiegeln im Blut einhergehen, sind für schwerwiegende Störungen mitursächlich.

Zur Senkung des Cholesterin- und/oder Neutralfettspiegels im menschlichen Blut sind schon zahlreiche pharmazeutische Produkte bekannt wie z.B. Lipostabil® oder andere lipidsenkende Pharmaka, die zur Senkung des Cholesterinspiegels insbesondere 2 - (4 - Chlorphenoxy) - 2 - methylpropionsäureäthylester (Clofibrat) als Wirkstoff enthalten. Wie literaturbekannt, hat das Clofibrat schwere Nachteile (vgl. z.B. British Heart Journal 1978, 40, S. 1069—1118). Häufig beobachtet man aber dazu noch, daß mit solchen Produkten eine nur unzureichende Senkung eines hohen Cholesterinspiegels mit Werten bis zu 500 mg % erreicht wurde oder nach einer gewissen Behandlungszeit in anderer Beziehung Therapieresistenz eintrat, und dies trotz gleichzeitiger Einhaltung einer fettarmen Diät. Bei gleichzeitig überhöhten Neutralfettblutspiegeln mußten Kombinationspräparate eingesetzt werden, die Nikotinsäurederivate wie $\beta$-Pyridylcarbinol-Hydrogentartrat oder Inositolnikotinat enthielten.

Überraschenderweise wurde gefunden, daß mit den erfindungsgemäß verwendeten Wirkstoffen der erhöhte Cholesterinspiegel und gleichzeitig ein erhöhter Neutralfettspiegel oder auch nur ein überhöhter Blutfettspiegel ohne jede Nebenwirkung rasch in den normalen Bereich gesenkt oder eine Therapieresistenz bei zu hohem Cholesterinspiegel wirkungsvoll überwunden werden kann, und zwar selbst wenn man eine fettarme Diät überhaupt nicht oder nicht streng durchführt.

Die erfindungsgemäß verwendeten Wirkstoffe stellen an sich bekannte Produkte dar, die bischer zur Verhinderung von Schimmelwachstum auf Fleischprodukten verwendet wurden (vgl. z.B. DOS 1 767 439 und "Die Fleischwirtschaft", 1968, 1594 bis 1596; 1971, 517 bis 522 und 1972, 816 bis 818). Die erfindungsgemäß verwendeten Wirkstoffe stellen ein vollständig acetyliertes destilliertes, aus Schweineschmalz, Baumwellsamenöl oder teilweise hydriertem Pflanzenöl hergestelltes Monoglycerid oder Gemische mehrerer solcher Monoglyceride dar, d.h. ein acetyliertes Monoglycerid, bei dem die Verteilung der Kohlenstoffzahl in dem von Acetyl verschiedenen Acylrest und die Verteilung der gesättigten und gegebenenfalls ungesättigten Acylreste derjenigen in den genannten Fettausgangsprodukten Schweineschmalz, Baumwollsamenöl und teilweise hydriertem Pflanzenöl entspricht. Daher ist auch die Verwendung eines im wesentlichen entsprechenden Gemisches aus den synthetisch hergestellten einzelnen Diacetylmonoglyceriden mitumfaßt. Bevorzugt sind aber die aus den natürlichen Fettstoffen hergestellten acetylierten Monoglyceride. Bevorzugt sind solche Monoglyceride, wie sie aus Schweineschmalz hergestellt werden.

Es ist bekannt, Partialglyceride aus natürlichen Fettstoffen mit niederen Fettsäuren wie Essigsäure zu verestern. Die erfindungsgemäß als Wirkstoff verwendeten vollständig acetylierten Monoglyceride entsprechen dabei der folgenden Formel

$$\begin{array}{l} CH_2—O—R_1 \\ | \\ CH—O—R_2 \\ | \\ CH_2—O—R_3 \end{array}$$

worin einer der Reste $R_1$, $R_2$ und $R_3$ der Rest einer gesättigten oder olefinisch ungesättigten Fettsäure mit 12 bis 22 Kohlenstoffatomen und die beiden anderen Reste Acetylgruppen sind.

Im allgemeinen stellt der Rest der Fettsäure mit 12 bis 22 Kohlenstoffatomen ein Gemisch dar, wie es in den eingangs genannten natürlichen Fetten vorkommt. Die hauptsächlichsten Fettsäurereste

sind dabei die Reste der Stearinsäure, der Palmitinsäure und der Olsäure. Die genannten Produkte sind im Handel erhältlich.

Die Herstellung der destillierten acetylierten Monoglyceride, die erfindungsgemäß als solche erfindungsgemäße pharmazeutische Präparate sind oder im Gemisch mit üblichen Trägerstoffen oder Verdünnungsmitteln die Wirkstoffe der erfindungsgemäßen pharmazeutischen Präparate sind, erfolgt in bekannter Weise durch eine Umesterung eßbarer Fette mit Glycerintriacetat (Triacetin) in Gegenwart von Katalysatoren mit einer folgenden Molekulardestillation; die Monoglyceride können ebenfalls in bekannter Weise auch durch eine unmittelbare Acetylierung eßbarer Monoglyceride mit Essigsäureanhydrid, und zwar ohne Anwendung von Katalysatoren und einer molekularen Destillation hergestellt werden, wobei gegebenenfalls Essigsäure, Essigsäureanhydrid und Triacetin durch eine Vakuumdestillation entfernt werden.

Destilliertes, aus bestem Schweineschmalz gewonnenes, vollständig acetyliertes Monoglycerid (nachfolgend Monoglycerid SCH bezeichnet) hat dabei die folgenden Eigenschaften:

| | |
|---|---|
| Physikalische Eigenschaften: | |
| Aussehen | klar, fast farblos, flüssig |
| Erstarrungspunkt | bei etwa +8°C |
| Refraktionsindex | 1447 (40°C) |
| | 1443 (50°C) |
| Viskosität | |
| bei +20°C | 50 cP |
| bei +50°C | 19 cP |
| Spezifisches Gewicht | |
| bei +20°C | 0,99 |
| bei +50°C | 0,96 |
| Chemische Eigenschaften: | |
| Jodzahl | 42 |
| Verseifungszahl | 380 |
| % Monoglycerid | 0 bis 2 |
| Reichert-Meissel-Zahl | 145 |
| Säurezahl | weniger als 4 |
| Peroxidzahl | weniger als 2 |

Destilliertes, aus teilweise hydriertem Pflanzenöl gewonnenes, voll-ständig acetyliertes Monoglycerid (nachfolgend Monoglycerid PF bezeichnet) hat die folgenden Eigenschaften:

| | |
|---|---|
| Physikalische Eigenschaften: | |
| Aussehen | klar, fast farblose Flüssigkeit |
| Erstarrungspunkt | etwa 7°C |
| Refraktionsindex | 1447 (40°) |
| Viskosität | |
| bei +20°C | 56 cP |
| bei +50°C | 19 cP |
| Spezifisches Gewicht | |
| bei +20°C | 0,98 |
| bei +50°C | 0,96 |
| Chemische Eigenschaften: | |
| Jodzahl | 44 |
| Verseifungszahl | 380 |
| % Monoglycerid | 0 bis 2 |
| Reichert-Meissel-Zahl | 146 |
| Säurezahl | weniger als 4 |

Destilliertes, aus Baumwollsamenöl gewonnenes vollständig acetyliertes Monoglycerid (nachfolgend Monoglycerid BW bezeichnet) hat die folgenden Eigenschaften:

Physikalische Eigenschaften:

| | |
|---|---|
| Aussehen | klar, sehr schwachgel b-gefärbte Flüssigkeit |
| Erstarrungspunkt | bei etwa +1 °C |
| Refraktionsindex | 1451 (40°C) |
| Viskosität | |
| bei +20°C | 47 cP |
| bei +50°C | 18 cP |
| Spezifisches Gewicht | |
| bei +20°C | 0,98 |
| bei +50°C | 0,96 |

Chemische Eigenschaften:

| | |
|---|---|
| Jodzahl | 70 |
| Verseifungszahl | 380 |
| % Monoglycerid | 0 bis 2 |
| Reichert-Meissel-Zahl | 145 |
| Säurezahl | weniger als 4 |

Die erfindungsgemäß verwendeten Monoglyceride gelangen im wesentlichen als solche, d.h. in Reinform zur Anwendung, wobei die Produkte in einer Dosierung von 1 bis 15 ccm pro Verabreichung je nach dem zeitlichen Abstand der Verabreichungen voneinander und dem Cholesterinblutspiegel des zu behandelnden Patienten verabreicht werden.

Die erfindungsgemäß verwendeten Wirkstoffe können jedoch auch in anderer geeigneter Darreichungsform angewendet werden. Sie können auch diätetischen Nahrungsmitteln zugemischt und in dieser Form verabreicht werden. Weiterhin lassen sich leicht mit den in lipidsenkenden Produkten üblicherweise eingesetzten Vitaminen und/oder Phospholipiden und anderen Zusatzstoffen hierfür wie Desoxycholsäure kombinieren. Insbesondere die Kombination mit den so verwendeten Vitaminen A, D und/oder E und/oder den Phospholipiden ist darum leicht möglich, weil diese sich hierin lösen.

Anwendungsbeispiele

1. Eine 50-jährige Patientin, die u.a. unter Hyperlipidaemie 2b leidet, hatte zu Beginn der Behandlung (1. Juni) einen Cholesterinblutspiegel von 354 mg/dl und einen Neutralfettblutspiegel von 122 mg/dl. Sie erhielt 10 Tage 3x1 Teelöffel Monoglycerid SCH und dann täglich 3x1/2 Teelöffel. Nach 37 Tagen (7. Juli) betrug der Cholesterinblutspiegel 307 mg/dl, der Neutralfettblutspiegel 107 mg/dl, nach weiteren 2 Monaten (8. September) 231 bzw. 136 mg/dl und nach einem weiteren Monat (5. Oktober) bei täglich 3x1/2 Teelöffel 254 bzw. 107 mg/dl. Die Dosis wurde auf die Initialdosierung erhöht. Etwa 1 Monat danach (2. November) bzw. 3 Monate danach (11. Januar) betrugen Cholesterin- und Neutralfettblutspiegel 233 bzw. 106 und 200 bzw. 104 mg/dl. Das Öl war am 15. Dezember abgesetzt worden. Trotz der Weihnachtsfeiertage blieben die Blutspiegelwerte im absoluten physiologischen Bereich. Das Öl hatte somit auch einen stabilisierenden Effekt.

2. Ein 73-jähriger, unter Diabetes mellitus, Aortensklerose, Coronarinsuffizienz und Hyperlipidaemie 2b leidender Patient hatte zu Beginn der Therapie (13. Januar) einen Cholesterinspiegel von 426,6 mg/dl und einen Neutralfettblutspiegel von 169 mg/dl. Zusätzlich zu Diät erhielt er 750 mg Clofibrat pro Tag. Nach etwa 3 Monaten (21.4.) betrugen die Werte 418 bzw. 211 mg/dl.

Die Behandlung wurde zusätzlich zu Diät mit einem 500 mg Clofibrat und 300 mg Inositolnicotinat pro Kapsel enthaltendem Präparat (3x1 Kapsel pro Tag) fortgesetzt. Nach 1 Monat (26. Mai) betrugen Cholesterin- bzw. Neutralfettblutspiegel 318 bzw. 149 mg/dl. Das Kombinationspräparat mußte wegen Auftretens von generalisierter Pruritus abgesetzt werden. Es wurde weiterbehandelt mit dem Monoglycerid SCH, das 10 Tage in einer Dosis von 3x1 Teelöffel pro Tag und hiernach in einer Dosis von 3x1/2 Teelöffel pro Tag verabreicht wurde. Die Blutfettkurven fielen steil ab Nach 1 Monat (30. Juni) betrugen Cholesterin- bzw. Neutralfettblutspiegel 212 bzw. 107 mg/dl. Es traten keinerlei Zeichen von Unverträglichkeit oder Nebenwirkung auf.

3. Ein 73-jähriger Patient, der zusätzlich zu Hyperlipidaemie 2b unter hypersekretorischer Gastropathie, Gärungsdyspepsie, Coronarinsuffizienz und essentieller Hypertonie litt, zeigte zu Beginn der Behandlung einen Cholesterinblutspiegel von 280 mg/dl und einen Neutralfettblutspiegel von 343,2 mg/dl. Diät alleine konnte keine Besserung bringen. Nach 8 Monaten betrugen Cholesterin- bzw. Neutralfettblutspiegel 292 bzw. 147 mg/dl. Es wurde 6 Monate Diät gehalten und gleichzeitig ein 500 mg Clofibrat und 25 mg β-Pyridylcarbinol-Hydrogentartrat pro Kapsel enthaltendes Kombinationspräparat in der vom Hersteller empfohlenen Dosierung verabreicht. Nach 6 Monaten betrug der Cholesterin- bzw. Neutralfettblutspiegel 406 bzw. 201 mg/dl. Akute hypertone Krisen und pectanginöse Beschwerden traten nach wie vor auf. Die hohen Werte erklären sich möglicherweise gegenregulatorisch dadurch, daß der Patient die Therapie kurz vor diesem Untersuchungsdatum spontan abgesetzt hatte. Die Fortsetzung der Therapie erfolgte durch Verabreichung des Monoglycerids SCH für 10 Tage in einer Dosierung von 3×1 Teelöffel und sodann 3×1/2 Teelöffel. Nach etwas mehr als 1 Monat betrugen Cholesterin- bzw. Neutralfettspiegel 254 bzw. 96 mg/dl. Die Therapie wurde mit dem Monoglycerid SCH in einer Dosierung von 3×1/2 Teelöffel fortgesetzt. Nach weiteren 2 bzw. 5 Monaten betrugen Cholesterin- bzw. Neutralfettblutspiegel 216 bzw. 94 und 221 bzw. 109 mg/dl. Die systematische Therapie mit dem Monoglycerid SCH brachte den Erfolg. Sowohl Cholesterin als auch Neutralfett fielen um 45,57 bzw. 45,77% in den absoluten Normbereich ab. Es wurden keine Zeichen von Unverträglichkeit oder Nebenwirkungen beobachtet. Der Patient ist seit Herbst 1978 beschwerdenfrei und meldet allgemeines Wohlbefinden.

**Patentansprüche für die Vertragsstaaten BE, CH, FR, GB, IT, LU, NL, SE**

1. Vollständig acetylierte Monoglyceride der allgemeinen Formel

$$\begin{array}{l} CH_2—O—R_1 \\ | \\ CH—O—R_2 \\ | \\ CH_2—O—R_3 \end{array}$$

worin einer der Reste $R_1$, $R_2$ und $R_3$ der Rest einer gesättigten oder olefinisch ungesättigten Fettsäure mit 12 bis 22 Kohlenstoffatomen und die beiden anderen Reste Acetylgruppen sind, oder eines Gemisches aus mehreren solcher acetylierter Monoglyceride zur Verwendung zur Senkung von überhöhten Cholesterinspiegeln und/oder Neutralfettspiegeln im Blut von Menschen und/oder zur Überwindung von Therapieresistenz von Menschen mit erhöhtem Cholesterin- und/oder Neutralfettblutspiegel gegen übliche pharmazeutische Produkte zur Senkung von überhöhtem Cholesterin- und/oder Neutralfettspiegel im Blut von Menschen.

2. Vollständig acetylierte Monoglyceride gemäß Anspruch 1, dadurch gekennzeichnet, daß das oder die Monoglyceride ein vollständig acetyliertes destilliertes, aus Schweineschmalz, Baumwollsamenöl oder teilweise hydriertem Pflanzenöl hergestelltes Produkt ist.

3. Vollständig acetylierte Monoglyceride gemäß Anspruch 1 und 2, dadurch gekennzeichnet, daß das oder die Monoglyceride ein vollständig acetyliertes destilliertes, aus Schweineschmalz hergestelltes Produkt ist.

4. Pharmazeutische Präparate zur Verwendung zur Senkung von überhöhten Cholesterinspiegeln und/oder Neutralfettspiegeln im Blut von Menschen und/oder zur Überwindung von Therapieresistenz von Menschen mit erhöhtem Cholesterin- und/oder Neutralfettblutspiegel gegen übliche pharmazeutische Produkte zur Senkung von überhöhtem Cholesterin- und/oder Neutralfettspiegel im Blut von Menschen, dadurch gekennzeichnet, daß sie aus einem vollständig acetyliertem Monoglycerid der allgemeinen Formel

$$\begin{array}{l} CH_2—O—R_1 \\ | \\ CH—O—R_2 \\ | \\ CH_2—O—R_3 \end{array}$$

worin einer der Reste $R_1$, $R_2$ und $R_3$ der Rest einer gesättigten oder olefinisch ungesättigten Fettsäure mit 12 bis 22 Kohlenstoffatomen und die beiden anderen Reste Acetylgruppen sind, oder ein Gemisch aus mehreren solcher acetylierter Monoglyceride bestehen oder als pharmakologisch aktiven Bestandteil ein solches enthalten.

5. Pharmazeutische Präparate gemäß Anspruch 4, dadurch gekennzeichnet, daß der Wirkstoff ein vollständig acetyliertes destilliertes, aus Schweineschmalz, Baumwollsamenöl oder teilweise hydrierten Pflanzenöl hergestelltes Monoglycerid ist.

6. Pharmazeutische Präparate gemäß Anspruch 4 und 5, dadurch gekennzeichnet, daß der Wirkstoff ein vollständig acetyliertes destilliertes, aus Schweineschmalz hergestelltes Monoglycerid ist.

7. Pharmazeutische Präparate gemäß Ansprüchen 4—6, dadurch gekennzeichnet, daß sie das Monoglycerid in Kombination mit einem oder mehreren Vitaminen und/oder einem oder mehreren Phospholipiden und gegebenenfalls Desoxycholsäure oder einem Salz hiervon enthalten.

8. Diätetische Nahrungsmittel mit cholesterin- und/oder neutralfettblutspiegelsenkender Wirkung, dadurch gekennzeichnet, daß sie ein vollständig acetyliertes Monoglycerid der allgemeinen Formel

$$\begin{array}{l} CH_2—O—R_1 \\ | \\ CH—O—R_2 \\ | \\ CH_2—O—R_3 \end{array}$$

worin einer der Reste $R_1$, $R_2$ und $R_3$ der Rest einer gesättigten oder olefinisch ungesättigten Fettsäure mit 12 bis 22 Kohlenstoffatomen und die beiden anderen Reste Acetlygruppen sind, oder ein Gemisch aus mehreren solcher acetylierter Monoglyceride beigemischt enthalten.

9. Diätetische Nahrungsmittel gemäß Anspruch 8, dadurch gekennzeichnet, daß der Wirkstoff ein vollständig acetyliertes, destilliertes, aus Schweineschmalz, Baumwollsamenöl oder teilweise hydrierten Pflanzenöl hergestelltes Monoglycerid ist.

10. Diätetisches Nahrungsmittel gemäß Anspruch 8 und 9, dadurch gekennzeichnet, daß der Wirkstoff ein vollständig acetyliertes destilliertes, aus Schweineschmalz hergestelltes Monoglycerid ist.

**Patentansprüche fur den Vertragsstaat: AT**

1. Verfahren zur Herstellung pharmazeutischer Präparate zur Verwendung zur Senkung von überhöhten Cholesterinspiegeln und/oder Neutralfettspiegeln im Blut von Menschen und/oder zur Überwindung von Therapieresistenz von Menschen mit erhöhtem Cholesterin- und/oder Neutralfettblutspiegel gegen übliche pharmazeutische Produkte zur Senkung von überhöhtem Cholesterin- und/oder Neutralfettspiegel im Blut von Menschen, dadurch gekennzeichnet, daß man ein vollständig acetyliertes Monoglycerid der allgemeinen Formel

$$\begin{array}{l} CH_2—O—R_1 \\ | \\ CH—O—R_2 \\ | \\ CH_2—O—R_3 \end{array}$$

worin einer der Reste $R_1$, $R_2$ und $R_3$ der Rest einer gesättigten oder olefinisch ungesättigten Fettsäure mit 12 bis 22 Kohlenstoffatomen und die beiden anderen Reste Acetylgruppen sind, oder eines Gemisches aus mehreren solcher acetylierter Monoglyceride gegebenenfalls mit einem üblichen pharmazeutischen Trägerstoff und/oder Verdünnungsmittel vermischt und in eine übliche Dosierungseinheit bringt.

2. Verfahren gemäß Anspruch 1, dadurch gekennzeichnet, daß das oder die Monoglyceride ein vollständig acetyliertes destilliertes, aus Schweineschmalz, Baumwollsamenöl oder teilweise hydriertem Pflanzenöl hergestelltes Produkt ist.

3. Verfahren gemäß Anspruch 1 und 2, dadurch gekennzeichnet, daß das oder die Monoglyceride ein vollständig acetyliertes destilliertes, aus Schweineschmalz hergestelltes Produkt ist.

4. Verfahren zur Herstellung von diätetischen Nahrungsmitteln mit cholesterin- und/oder neutralfettblutspiegelsenkender Wirkung, dadurch gekennzeichnet, daß man ein vollständig acetyliertes Monoglycerid der allgemeinen Formel

$$\begin{array}{l} CH_2—O—R_1 \\ | \\ CH—O—R_2 \\ | \\ CH_2—O—R_3 \end{array}$$

worin einer der Reste $R_1$, $R_2$ und $R_3$ der Rest einer gesättigten oder olefinisch ungesättigten Fettsäure mit 12 bis 22 Kohlenstoffatomen und die beiden anderen Reste Acetylgruppen sind, oder ein Gemisch aus mehreren solcher acetylierter Monoglyceride mit üblichen Bestandteilen diätetischer Nahrungsmittel vermischt.

5. Verfahren gemäß Anspruch 4, dadurch gekennzeichnet, daß als Monoglycerid ein vollständig acetyliertes, destilliertes, aus Schweineschmalz, Baumwollsamenöl oder teilweise hydrierten Pflanzenöl hergestelltes Monoglycerid eingesetzt wird.

6. Verfahren gemäß Anspruch 4 und 5, dadurch gekennzeichnet, daß als Monoglycerid ein vollständig acetyliertes, destilliertes, aus Schweineschmalz hergestelltes Monoglycerid verwendet wird.

**Revendications pour les Etats contractants: BE, CH, FR, GB, IT, LU, NL, SE**

1. Monoglycérides entièrement acétylés de formule générale:

$$\begin{array}{l} CH_2—O—R_1 \\ | \\ CH—O—R_2 \\ | \\ CH_2—O—R_3 \end{array}$$

où l'un des radicaux $R_1$, $R_2$ et $R_3$ est le radical d'un acide gras saturé ou oléfiniquement insaturé en $C_{12}$ à

$C_{22}$ et les deux autres radicaux sont des groupes acétyle, ou mélange de plusieurs monoglycérides acétylés de ce genre aux fins d'application pour abaisser les niveaux de cholestérol et/ou de graisses neutres excessifs dans le sang chez l'homme et/ou pour surmonter une résistance thérapeutique, chez des personnes ayant un niveau sanguin accru de cholestérol et/ou de graisse neutre, aux produits pharmaceutiques habituels visant à diminuer l'excès de niveau de cholestérol et/ou de graisses neutres dans le sang chez l'homme.

2. Monoglycérides entièrement acétylés selon la revendication 1, caractérisés en ce que le ou les monoglycéride(s) est (sont) un produit distillé entièrement acétylé, préparé à partir de saindoux, d'huile de coton ou d'huile végétale partiellement hydrogénée.

3. Monoglycérides entièrement acétylés selon les revendications 1 et 2, caractérisés en ce que le ou les monoglycéride(s) est (sont) un produit distillé entièrement acétylé préparé à partir de saindoux.

4. Préparations pharmaceutiques aux fins d'application à l'abaissement de l'excés de niveau de cholestérol et/ou de graisses neutres dans le sang chez l'homme et/ou pour surmonter une résistance thérapeutique, chez l'homme ayant un niveau sanguin accru de cholestérol et/ou de graisses neutres, aux produits pharmaceutiques habituels visant à abaisser l'excès de niveau de cholestérol et/ou de graisses neutres dans le sang chez l'homme, caractérisées en ce qu'elles se composent d'un monoglycéride entièrement acétylé de formule générale

$$\begin{array}{l} CH_2—O—R_1 \\ | \\ CH—O—R_2 \\ | \\ CH_2—O—R_3 \end{array}$$

où l'un des radicaux $R_1$, $R_2$ et $R_3$ est la radical d'un acide gras saturé ou oléfiniquement insaturé en $C_{12}$ à $C_{22}$ et les deux autres radicaux sont des groupes acétyle, ou d'un mélange de plusieurs monoglycérides acétylés de ce genre ou en ce qu'elles en contiennent comme composant(s) pharmacologiquement actifs.

5. Préparations pharmaceutiques selon la revendication 4, caractérisées en ce que la substance active est un monoglycéride distillé entièrement acétylé, préparé à partir de saindoux, d'huile de coton ou d'huile vététale partiellement hydrogénée.

6. Préparations pharmaceutiques selon les revendications 4 et 5, caractérisées en ce que la substance active est un monoglycéride entièrement acétylé distillé, préparé à partir de saindoux.

7. Préparations pharmaceutiques selon les revendications 4—6, caractérisées en ce qu'elles contiennent le monoglycéride en combinaison avec une ou plusieurs vitamines et/ou un ou plusieurs phospholipides et éventuellement l'acide désoxycholique ou un de ses sels.

8. Aliments diététiques à action d'abaissement du niveau sanguin de cholestérol et/ou de graisses neutres, caractérisés en ce qu'ils contiennent un monoglycéride entièrement acétylé de formule générale

$$\begin{array}{l} CH_2—O—R_1 \\ | \\ CH—O—R_2 \\ | \\ CH_2—O—R_3 \end{array}$$

où l'un des radicaux $R_1$, $R_2$ et $R_3$ est le radical d'un acide gras saturé ou oléfiniquement insaturé en $C_{12}$ à $C_{22}$ et les deux autres radicaux sont des groupes acétyle, ou un mélange de plusieurs monoglycérides acétylés de ce genre.

9. Aliments diététiques selon la revendication 8, caractérisés en ce que la substance active est un monoglycéride entièrement acétylé, distillé, préparé à partir de saindoux, d'huile de coton ou d'huile végétale partiellement hydrogénée.

10. Aliment diététique selon les revendications 8 et 9, caractérisé en ce que la substance active est un monoglycéride entièrement acétylé, distillé, préparé à partir de saindoux.

**Revendications pour l'Etat contractant: AT**

1. Procédé de fabrication d'une préparation pharmaceutique aux fins d'application à l'abaissement de l'excès de niveau de cholestérol et/ou de graisses neutres dans le sang chez l'homme et/ou pour surmonter une résistance thérapeutique, chez l'homme ayant un niveau sanguin accru de cholestérol et/ou de graisses neutres, aux produits pharmaceutiques habituels visant à abaisser l'excès de niveau de cholestérol et/ou de graisses neutres dans le sang chez l'homme, caractérisé en ce que l'on mélange un monoglycéride entièrement acétylé de formule générale

$$
\begin{array}{l}
CH_2—O—R_1 \\
| \\
CH—O—R_2 \\
| \\
CH_2—O—R_3
\end{array}
$$

où l'un des radicaux $R_1$, $R_2$ et $R_3$ est le radical d'un acide gras saturé ou oléfiniquement insaturé en $C_{12}$ à $C_{22}$ et les deux autres radicaux sont des groupes acétyle, ou un mélange de plusieurs monoglycérides acétylés de ce type, éventuellement avec un support et/ou un diluant habituellement pharmaceutiquement acceptable, et en ce qu'on l'amène sous la forme d'une unité de dosage habituelle.

2. Procédé selon la revendication 1, caractérisé en ce que le ou les monoglycérides est ou sont constitué(s) par un composé monoglycéride distillé entièrement acétylé, préparé à partir de saindoux, d'huile de coton ou d'huile végétale partiellement hydrogénée.

3. Procédé selon l'une des revendications 1 et 2, caractérisé en ce que le ou les monoglycérides est ou sont constitué(s) par un composé monoglycéride distillé entièrement acétylé, préparé à partir de saindoux.

4. Procédé de préparation d'aliments diététiques à action d'abaissement du niveau sanguin de ch lestérol et/ou de graisses neutres, caractérisé en ce qu'on mélange un monoglycéride entièrement acétylé de formule générale

$$
\begin{array}{l}
CH_2—O—R_1 \\
| \\
CH—O—R_2 \\
| \\
CH_2—O—R_3
\end{array}
$$

où l'un des radicaux $R_1$, $R_2$ et $R_3$ est le radical d'un acide gras saturé ou oléfiniquement insaturé en $C_{12}$ à $C_{22}$ et les deux autres radicaux sont des groupes acétyle, ou un mélange de plusieurs monoglycérides acétylés de ce type, avec un des constituants habituels d'aliments diététiques.

5. Procédé selon la revendication 4, caractérisé en ce qu'on utilise un monoglycéride distillé entièrement acétylé, préparé à partir de saindoux, d'huile de coton ou d'huile végétale partiellement hydrogénée.

6. Procédé selon la revendication 4 ou 5, caractérisé en ce qu'on utilise un monoglycéride distillé entièrement acétylé, préparé à partir de saindoux.

**Claims for the Contracting states: BE, CH, FR, GB, IT, LU, NL, SE**

1. Wholly acetylated monoglycerides of general formula

$$
\begin{array}{l}
CH_2—O—R_1 \\
| \\
CH—O—R_2 \\
| \\
CH_2—O—R_3
\end{array}
$$

wherein one of the radicals $R_1$, $R_2$ and $R_3$ is the radical of a saturated or olefinic-unsaturated fatty acid with 12 to 22 carbon atoms and wherein both other radicals are acetyl groups, or a mixture of several such acetylated monoglycerides for use for lowering of raised levels of cholesterol and/or neutral fats in human blood and/or for overcoming of therapy resistance of human beings suffering from raised levels of cholesterol and/or neutral fats to common pharmaceutical products for lowering of raised levels of cholesterol and/or neutral fats in human blood.

2. Wholly acetylated monoglycerides according to claim 1, characterized in that the monoglyceride(s) is a wholly acetylated distilled product obtained from lard, cotton seed oil or partially hydrogenated vegetable oil.

3. Wholly acetylated monoglycerides according to claims 1 and 2, characterized in that the monoglyceride(s) is a wholly acetylated distilled product obtained from lard.

4. Pharmaceutical compositions for use for lowering of raised levels of cholesterol and/or neutral fats in human blood and/or for overcoming of therapy resistance of human beings suffering from raised levels of cholesterol and/or neutral fats to common pharmaceutical products for lowering of raised levels of cholesterol and/or neutral fats in human blood, characterized in that they consist of a wholly acetylated monoglyceride of general formula

$$
\begin{array}{l}
CH_2—O—R_1 \\
| \\
CH—O—R_2 \\
| \\
CH_2—O—R_3
\end{array}
$$

wherein one of the radicals $R_1$, $R_2$ and $R_3$ is the radical of a saturated or olefinic-unsaturated fatty acid with 12 to 22 carbon atoms and wherein both other radicals are acetyl groups, or of a mixture of several such acetylated monoglycerides or contain such one as pharmaceutically active constituent.

5. Pharmaceutical compositions according to claim 4, characterized in that the active constituent is a wholly acetylated distilled monoglyceride obtained from lard, cotton seed oil or partially hydrogenated vegetable oil.

6. Pharmaceutical compositions according to claims 4 and 5, characterized in that the active constituent is a wholly acetylated distilled monoglyceride obtained from lard.

7. Pharmaceutical compositions according to claims 4—6, characterized in that they contain the monoglyceride combined with one or more vitamines and/or one or more phospholipides and optionally desoxycholic acid or a salt thereof.

8. Dietetic foodstuffs with an activity for lowering the levels of cholesterol and/or neutral fats in blood, characterized in that they contain a wholly acetylated monoglyceride of general formula

$$\begin{array}{l} CH_2—O—R_1 \\ | \\ CH—O—R_2 \\ | \\ CH_2—O—R_3 \end{array}$$

wherein one of the radicals $R_1$, $R_2$ and $R_3$ is the radical of a saturated or olefinic-saturated fatty acid with 12 to 22 carbon atoms and wherein both other radicals are acetyl groups, or a mixture of several such acetylated monoglycerides mixed in.

9. Dietetic foodstuffs according to claim 8, characterized in that the active constituent is a wholly acetylated distilled monoglyceride obtained from lard, cotton seed oil or partially hydrogenated vegetable oil.

10. Dietetic foodstuffs according to claims 8 and 9, characterized in that the active constituent is a wholly acetylated distilled monoglyceride obtained from lard.

**Claims for the Contracting state AT**

1. A process for preparing pharmaceutical compositions for use for lowering of raised levels of cholesterol and/or neutral fats in human blood and/or for overcoming of therapy resistance of human beings suffering from raised levels of cholesterol and/or neutral fats in blood to common pharmaceutical products for lowering of raised levels of cholesterol and/or neutral fats in human blood, characterized in that a wholly acetylated monoglyceride of general formula

$$\begin{array}{l} CH_2—O—R_1 \\ | \\ CH—O—R_2 \\ | \\ CH_2—O—R_3 \end{array}$$

wherein one of the radicals $R_1$, $R_2$ and $R_3$ is the radical of a saturated or olefinic-unsaturated fatty acid with 12 to 22 carbon atoms and wherein both other radicals are acetyl groups, or a mixture of several such acetylated monoglycerides is mixed optionally with a common pharmaceutical carrier and/or diluent and formulated to a common dose unit.

2. A process according to claim 1, characterized in that the monoglyceride(s) is a wholly acetylated distilled product obtained from lard, cotton seed oil or partially hydrogenated vegetable oil.

3. A process according to claim 1 and 2, characterized in that the monoglyceride(s) is a wholly acetylated distilled product, obtained from lard.

4. A process for preparing dietetic foodstuffs having an activity for lowering the levels of cholesterol and/or neutral fats in blood, characterized in that a wholly acetylated monoglyceride of general formula

$$\begin{array}{l} CH_2—O—R_1 \\ | \\ CH—O—R_2 \\ | \\ CH_2—O—R_3 \end{array}$$

wherein one of the radicals $R_1$, $R_2$ and $R_3$ is the radical of a saturated or olefinic-unsaturated fatty acid with 12 to 22 carbon atoms and wherein both other radicals are acetyl groups, or a mixture of several such acetylated monoglycerides is mixed with common constituents of dietetic foodstuffs.

5. A process according to claim 4, characterized in that a wholly acetylated distilled

monoglyceride, obtained from lard, cotton seed oil or partially hydrogenated vegetable oil, is used as monoglyceride.

6. A process according to claim 4 and 5, characterized in that a wholly acetylated distilled product, obtained from lard, is used as monoglyceride.